# EUROPEAN PATENT APPLICATION

(11) **EP 4 628 144 A1**
(43) Date of publication of application: **08.10.2025**
(21) Application number: 24168865.4
(22) Date of filing: 05.04.2024
(51) Int. Cl.: A61M 39/10, A61M 39/18

(54) **GENDERLESS ASEPTIC CONNECTING DEVICE FOR FLUID CONNECTION AND CONNECTION ARRANGEMENT USING A PAIR OF CONNECTING DEVICES**

(71) Applicant: SARTORIUS STEDIM FMT SAS, 13400 Aubagne (FR)
(72) Inventor: Delasalle, Brice, 13400 AUBAGNE (FR); Blake, Florian, 13400 AUBAGNE (FR)
(74) Representative: Plasseraud IP

(57) **Abstract**

The connector (1) has a body (2) provided with a flange (3), forming a central opening (O) between a female member (31) and a male member (32). The planar flange face (F3) has an axial access (30) of the female member and the male member (32) protrudes axially from the flange face. A membrane (M) removably attached to the connector covers this face to seal the opening, so that a genderless connecting device is obtained, allowing mating engagement of the connector (1) with an identical complementary connector (101). A clipping interface forming the female member allows locking a pre-coupled state of these connectors (1, 101) with each membrane still present. The flange of the connector (1) houses a slider (10) adapted to contact the mating insert (104) of the male member engaged through the axial access (30). The slider allows locking and/or unlocking a final position of the insert.

## Description

### FIELD OF THE INVENTION

The instant disclosure generally relates to fluid management systems in the field of biopharmaceutical applications, including locking components for efficiency of fluid circulation, and more particularly to a fluid handling coupling or connector that can allow a quick connection, while being genderless, and to an aseptic coupling arrangement using a pair of such connectors.

### TECHNICAL BACKGROUND

In the pharmaceutical, chemical field, that uses tubing and assembling of components in the processing and transport for fluids (such as biopharmaceutical fluids), there is often a need for aseptic connection, at least for the first connection. Hoses carrying biopharmaceutical fluid may be commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field.

Aseptic coupling devices can be used to connect two or more sterilized pathways. For example, aseptic connectors can be used to couple a fluid pathway from a first piece of processing equipment or container to a fluid pathway from a second piece of processing equipment or container to establish a sterile pathway for fluid transfer therebetween.
Generally, aseptic connectors require a dry connection obtained using clamping devices placed upstream of the aseptic connectors so that the aseptic connectors are kept free of fluid while the connection between the aseptic coupling devices is made. After establishing the sterile connection between the connectors, the clamping devices are removed to allow fluid to flow through the aseptic connectors.

Current connectors may be of complex conception, requiring assembling steps or handling operations before practically obtaining the fluid connection. Some connection systems require an additional interface forming a female interface for the two devices forming a male connector. Other connections systems imply a combination of an axial coupling and rotation of sleeve or collar to lock the connection.

In biotechnology and bioprocessing field, vessels have new construction adapted for single use applications. There is a need for minimizing complexity and amount of plastic material of aseptic connectors, while ensuring sterility and robustness of the connections, typically using shieldable flanged connecting devices that have each a removable membrane (to shield the proximal/front opening of the connector).

Document US 2003/0030272 discloses a disposable aseptic connection system that is a disposable connection. This kind of connector has an orientation-specific latching means provided with flexible clips, which are quite fragile parts. If a temporary connection is wanted, disconnection requires complicated handling to separate the coupling parts of the connectors. And, in contrast, if a permanent connection is wanted, an additional clamping assembly must be assembled, using stainless steel sanitary clamps. More generally, there is still room for improvement regarding constructions of fluid connectors, in a way optimizing the way of operating with such fluid connectors, in aseptic conditions and/or in user-friendly conditions.

### OBJECTS AND SUMMARY

For improving situation, embodiments of the invention provide a connecting device for fluid connection, having a connector that is genderless, the connecting device comprising:
- a tubular body extending around a longitudinal axis of the connector and delimiting an interior space (for allowing fluid passage), the tubular body having an opening at a front of the tubular body where the interior space axially opens;
- a flange formed peripherally on the tubular body around the interior space, the flange comprising a female connecting member that is provided with a locking region and an axial access, the axial access preferably opening at the front of the tubular body, and a male connecting member protruding axially from a flange front face to provide a first insert having a fastening region;
- a sealing element providing an annular contact surface at the flange front face, radially interposed between the opening on the one hand, and the axial access and the male connecting member on the other hand; and
- a slider mounted on the flange to be movable relative to the flange, along a direction transverse to the longitudinal axis;
wherein the slider is a functional part of the female connecting member (for instance interfering or co-acting with the axial access), to modify the locking region provided with a retaining surface, the axial access allowing insertion of a second insert, of same structure as the first insert and thus also having a fastening region, the second insert being part of an identical complementary connector, genderless, for the fluid connection and being sized and shaped for engagement of the retaining surface with the fastening region of the second insert.

A slide can be formed, to facilitate disconnection and/or prevent any fragile part (for instance used in a clipping to lock the coupling) to be exposed. The slider can be pushable or actuatable, to be able to change the configuration of the locking region, depending on position of the slider with respect to the axial access. The slide, arranged in a housing of the flange, can extend generally within a transverse plane perpendicular to the longitudinal axis. The connecting device is genderless, due to the structure at the front of the flange, and may be provided without any piece rotating around the longitudinal, typically with no rotation gesture required once the connecting device is facing a compatible connecting device.

The connecting device may be provided with an aseptic attachment structure, which includes a connector front face and a membrane that is removably attached to the connector front face. The aseptic attachment structure can include a flange front face, the membrane being removably attached (directly) to the flange front face to seal the opening, typically with the membrane overlapping the sealing element.

The slide can be formed rearwardly, with respect to the membrane or the flange front face, while the fastening region of the first insert can typically extend, axially, at a given distance from the flange front face. This given distance (axial distance) can be more than axial distance, in the female connecting member, between the front face and the retaining surface.

The connecting device advantageously provides an integration of the flexible (fragile) parts in a housing or slide for the slider. The connecting device, genderless and user-friendly, can satisfy all the needs of a modular aseptic connection and possibly disconnection, with a peel-off membrane, an axial mating and sealing direction and using a clipping or similar latching that can be realized in an inner locking region, thus preventing any fragile parts to be broken. The flange can form a slide for the slider that is at least one amongst a locking slider and an unlocking slider of the female connecting member.

Such kind of connecting device thus combines ability for aseptic connection, using the membrane of the aseptic attachment structure to initially release the opening/front opening once the mechanical coupling is obtained (with each insert received in a corresponding connection axial access), and ease of handling with simple gestures. Geometry of the connector is compatible with implementation of a securing clip that can be either permanently snapping or can be reopenable, this securing clip remaining outside the fluid pathway, thus not breaching the fluid tightness. In some non-limiting options, an outer clipping ring or actuator can be carried by the flange, forming the locking slider with or without button, with or without elastic return part.

In some embodiments, the flange may have two opposite outer sides that are parallel sides, possibly extending substantially rectilinear. The slider may be a single piece or any suitable assembly, movable parallel to the two opposite outer sides, transversely and perpendicular to the membrane sealing the opening.

The external actuator can be mounted slidably, covering the two opposite outer sides that guide two parallel arms of the slider. A pushing surface may be provided in a piece or assembly forming the external actuator, with the pushing surface extending transverse to the two parallel arms.
Whatever the shape of the external actuator and the number of piece(s) involved, the external actuator may be operatively coupled to the latching means so that, when the connector is coupled to another connector (analogous connector or identical connector with an interlocking at a first insert of the connector and at a second insert of the other connector, using the female connecting members and associated latches/latching means), a displacement of the external actuator causes release of the engagement between the latching means and the second insert, whereby the external actuator can unlock the fluid connection.

According to embodiments, one or more of the following features may be provided:
- the tubular body extends around the longitudinal axis between a first open end (forming rear end when considering the longitudinal axis extends along a rear-front direction) and a second open end.
- the tubular body is a single piece, including the flange.
- the tubular body is an assembly, including the flange and a tubular extension part (optionally forming a nozzle at a rear end), the tubular extension part being inserted inside or around a tubular fitting integral with the flange.
- the aseptic attachment structure includes the second open end, the membrane being removably attached to the tubular body to seal the opening provided at the second open end.
- the flange is integral with the tubular body and defines a flange outer circumference.
- a housing or peripheral region is delimited by an external side wall of the flange, the housing or peripheral region being provided with the locking region;
- the flange has an external side wall having a longitudinal extension (parallel to the longitudinal extension);
- a flange shoulder or front projection is provided, the slider being arranged to slide parallel to one side or two parallel sides of the flange shoulder.
- the tubular body is carrying the seal in a front groove interposed between the second open end and a flange front projection.
- an annular recess housing the latching means is delimited between the flange front projection and the external side wall.

According to a particular embodiment, the slider may be provided with an external actuator, typically stiff/rigid, which protrudes beyond or is covering (externally) at least one side of the flange. The slider and the external actuator can be part of a sliding component/piece that is slidably mounted relative to the flange. In some options, one amongst an internal part of the slider and the external actuator possibly comprises two sliding arms between which the female connecting member and the male connecting member extend (longitudinal extension of these connecting members, relative to the transverse extension of the sliding arms).

In some options, embodiments of the connecting device provide one or more of the following features:
- the first insert comprises an inserting tab that is shifted/offset on a first side relative to the longitudinal axis.
- the slider has an interfering portion, extending perpendicular to a sliding direction (possibly perpendicular to slider arms) and suitable to be engaged by the second insert, in particular on a region offset/shifted on a second side (opposite from the first side) relative to the longitudinal axis.
- the retaining surface is configured to axially retain the second insert at the fastening region thereof in a locking configuration of the fluid connection.
- in the first insert, the axial distance between the fastening region and the flange front face may be a given distance superior to distance between the retaining surface (typically arranged internally in the flange) and the flange front surface.
- a clipping is obtained when engaging the second insert with the retaining surface, while a clipping may also be obtained, simultaneously, when engaging the first insert with the retaining surface of the connector of the other connecting device.
- due to the slight excess of the given distance (axial distance) as compared to the positioning of the retaining surface, a gap exists in coupled state between the two frontal (also called proximal) front faces of the flanges, so that the membranes can be extracted through this gap, using simultaneously two pull portions of these membranes distributed in the two identical connecting devices.
- each membrane is thinner than a half size of the gap and/or can be pulled and slide within this gap, starting from a pre-coupled state with the two identical connecting devices coupled before any removal of membrane.
- the flange front face is planar/substantially planar.
- the male connecting member forms the only part of the connector protruding axially from the flange front face.
- the insert (first insert) may be a single protruding member of the connector, which protrudes from the front face with a protruding extension greater than a depth (or longitudinal extension) of the axial access provided by the female connecting member.
- a complementary of shape/circumference may be used for having the second insert filling the axial access.
- the insert may be part of a group of parallel inserts that either engage into a single opening or slot forming the axial access, or engage in different openings available on the flange (each of the different openings at the flange front face).
- the membrane is configured to be pulled radially along a pulling direction.
- the slider has two parallel arms (sliding arms) configured to slide along a direction that is perpendicular to the pulling direction for removing the membrane.

Possibly, the seals may of simple shape, for instance without requirement for any transverse portion protruding radially outward and/or externally covering the flange front face. Each of the seals may have an expanded state, to have as seal length that is superior to a depth of a front groove housing the corresponding seal, whereby the seals can be mutually in axial contact outside the grooves, within the gap. No rotation of the seals is required, once the first and second inserts are facing the corresponding axial accesses of the female connecting members.

In some embodiments, the slider may be movable parallel to the pulling direction for removing the membrane. For instance, the two parallel sliding arms can be configured to slide along a direction that is parallel to this pulling direction. Possibly, the slider is included in a separate piece, sliding inside a housing of the flange and adapted to unclip the second insert by pushing radially outward a clip, tongue or similar flexible member of the second insert. The slider forms an unlocking slider; for instance without any flexible part in the slider. In options, the slider is externally mounted on the flange and can be synchronized with a complementary slider, mounter on the connecting device, to allow a simultaneous sliding of the two sliders in response to a pushing action, whereby simultaneous unclipping of the first insert and the second insert can be obtained.

According to embodiments, the slider may unlock a locked state of the female connecting member, by actuating the slider directly or by actuating an external actuator that can possibly also be slidably mounted on the flange. For instance, an external actuator, distinct from the slider is provided with a pushing surface (or any suitable actuating portion, possibly pullable in a variant) extending outside the flange (with the external actuator being mounted on the flange, using a peripheral slide or covering the flange externally). The slider, which may be a single piece, includes the retaining surface and is actuatable/can be driven to cause the retaining surface to be displaced. The slider can be actuatable by the external actuator to unlock a locked state of the female connecting member when the connector is in connected state with the identical complementary connector.

According to embodiments, the slider in the female connecting member/interface is an annular piece, possibly configured as ring, which includes the retaining surface. The slider can be actuatable by a pushing action, for instance to allow an unlocking action allowing pulling/extracting the corresponding insert inserted via the axial access. The slider may be actuatable at a side of the slider that is away, preferably opposite, relative to the retaining surface to cause the retaining surface to be displaced radially outward, whereby the slider is a locking slider that is actuatable to unlock a locked state of the female connecting member when the connector is in connected state with the identical complementary connector.

The slider may form all or part of latching means for locking the coupling of the two connectors. The slider may include a piece distinct from the flange and can optionally be operable by a manual actuation using an external actuator or push-button, preferably arranged to externally cover the flange and/or radially protrude outward relative to the flange. The manual actuation may cause an interfering portion of the latching means/slider to be moved radially away from an axial direction passing through the axial access of the female connecting member.

In embodiments, the slider is a locking slider distinct from the tubular body and inserted in a housing of the flange. The second insert, formed in the complementary connector (suitable to be coupled to said connector for enabling the fluid connection) can be retained axially by the retained surface that is included on the locking slider. The locking slider can be:
- configured to perform the retaining of the second insert at the fastening region of the second insert.
- a slider arranged as a sliding clip, with an annular shape around the tubular body, the locking configuration being obtained when the second insert passing through the axial access is inserted in the female connecting member to have a second insert end extending axially beyond the retaining surface.
- provided with two parallel sliding arms distributed on two sides in the flange, possibly interconnected by an interfering portion that defines the retaining surface.
- slidable upon actuation of an external actuator provided with a pushing surface extending outside the flange.
- provided with the retaining surface, which is formed/provided in a portion of the locking slider, which is a portion radially movable toward the longitudinal axis, to engage the fastening region of the second insert.
- configured so that movement of the retaining surface toward the longitudinal axis is obtained automatically after insertion of the second insert, due to a return force exerted by a return part engaged against a shoulder or other rigid part of the tubular body, which may define an interior face of the housing.
- driven in position by an external actuator integral with the slider, possibly with a pushing of the external actuator moving the slider against the return force exerted by the return part, which is preferably included in the slider.
- of annular shape, preferably with two rectilinear outer edges (parallel) at the two parallel sliding arms, the locking slider extending around the tubular body and being retained in a housing (at least partly formed by the flange), possibly permanently retained using retaining tabs or lugs formed on the external side wall of the flange.
- axially inserted and mounted in the housing by a rear access facing in opposite direction from the flange front face.

In some options, the connector has an annular housing to house the locking slider, which is annular. The retaining surface can be provided at an end of the locking slider, at the opposite from another end coupled to or provided with an external actuator. Such another end is movable, typically pushable, along a first direction to allow a sliding of the locking slider. The external actuator, extending outside the housing, is movable, preferably pushable also along the first direction to allow a sliding of the locking slider. The pushing that is exerted on the pushable end, orientated along the first direction, may be an unlocking direction opposite to an elastic return force. The elastic return force may be provided by return means radially interposed between the tubular body and the end of the slider provided with/carrying the external actuator.

In the male connecting member, the first insert may be an inserting tab, possibly provided with a curved (slightly curved for instance) profile.
The fastening region may include an outer groove (facing radially outward), compatible with a U-section of a latch forming the slider (locking slider), with the locked position of the latch corresponding to an engagement of the base of the U-section inside the outer groove, possibly with a return-effect or similar constraint used to maintain the locked position. The locking slider may be movable, for instance slidable without any rotation. The slider may be actuatable like a guillotine, with the slider having an inner latch contact edge that can be concave to follow shape of curved outer groove formed on the inserting tab.

In embodiments of the connecting device with a flexible member provided in the first insert, one or more of the following features can be provided:
- the flexible member is forming at least one relief, while the second insert also has at least one relief in a flexible member.
- the flexible member allows a clipping, when engaged with the retaining surface.
- the locking configuration is unlockable by an unclipping action obtained by displacing the flexible member of the second insert toward a flexed position.
- the unclipping action requires an unlocking action performed by the slider, which is an unlocking slider.
- the flexible member has a protrusion extending in the slide in an engaged state (clipped state, for instance) of the corresponding insert.
- sliding of the slider in the slide allows the protrusion of the flexible member to be pushed (by a contacting portion of the slider) in a direction transverse to the sliding direction, so that the first insert can be disengaged by a pulling operation in a direction opposite to insertion direction for inserting the first insert in the female connecting member.
- the slider is an unlocking slider, partly covering the flange.
- a pushable portion of the slider (integral with the slider) is partly covering an external side wall of the flange.
- the slider is provided with a contacting portion, preferably arranged on one of the two parallel sliding arms of the sliders.
- in connected state (with two identical connectors interlocked with the first and second inserts engaged in the corresponding female connecting members), the contacting portion is configured to push or displace the at least one relief (forming a protrusion typically extending in the slide) of the second insert, in response to a pushing action exerted in the slider via an external actuator.
- the flexed position allows the second insert end to be moved back in the axial access.
- the unclipping action (unclipping obtained by displacing the flexible member of the second insert) is allowed as soon as the at least one relief has been displaced/moved out of the slide.
- the contacting portion is displaceable to reach an overlapping position to allow the disengagement of the second insert end.
- in the overlapping position, the contacting portion axially covers an inner face of the flange facing rearwardly and provided with a rear opening of the axial access.
- in the overlapping position, the contacting portion extends adjacent to and/or partly covers the rear opening.

Whatever the way the locking configuration is obtained, the slide can be arranged at a rear part of the female connecting member. In each connecting device, the closing member belonging to the membrane is covering the opening in the pre-coupled state. The pre-coupled state is a state, in which the locking configuration is established for axial retaining of two inserts distributed in two genderless connectors, so that two closing members are in an overlapped configuration and arranged to be in axial contact with each other. In the pre-coupled state (i.e. after properly inserting the second insert in the female connecting member), the closing member is adapted to be removed together with the other closing member, by using synchronizing means that are provided to mutually fasten the pull portions of the membranes or any other portion(s) of the membranes that extend beyond the flanges.

Whatever the way the locking configuration is obtained, the slide or a housing provided with the slide may open radially outward, through an external side wall of the flange, to allow positioning and/or controlling the slider. In some embodiments allowing pushing the slider, one or more of the following features are provided:
- the slider has a pushable portion provided outside the flange;
- the pushable portion is configured to disengage or drive disengagement of the retaining surface, upon a pushing action exerted on a pushing surface of the pushable portion.
- the pushing action exerted on a pushing surface allows a disconnection of the connector, preferably by removing an interfering portion (provided on the slider or on a flexible member of the insert) from a rectilinear trajectory for insertion and extraction of the insert (first insert) of male connecting member.
- the slider is configured to be transversely moved, along a first direction perpendicular to the longitudinal axis upon insertion of the second insert via the axial access,
- the slider is an locking slider that includes an elastic return part in radial contact with the tubular body.
- the elastic return part provides an elastic return force directed toward a second direction that is a sliding direction opposite to the first direction, whereby a clipping in an inserted state of the second insert in the female connecting member is allowed due to the elastic return force.
- before any connection, the slider is a locking slider clipped inside the flange, preferably to be permanently retained in the housing.
- the external side wall of the flange defines a circumference all around the slider.
- the external side wall of the flange forms a barrier configured to prevent the slider to be displaced along the slide by a centripetal pushing.
- the slider is a securing clip, of annular shape for instance, concealed by the external side wall and/or configured to perform a permanent clipping or snapping (snap lock, typically).
- the permanent clipping or snapping is obtained by axially retaining (permanently the second insert that is is also prevented to be rotated by the guiding channel delimiting the axial access in the female connecting member.

To obtain a connected state with the connector assembled (coupled) with a compatible connector of same structure, with the membranes ready/adapted to be removed to pass from a pre-coupled state to a coupled state, one or more of the following features are provided:
- the connector is a first aseptic coupling device for coupling to a second aseptic coupling device, consisting in the identical complementary connector and an associated membrane.
- the membrane of the first aseptic coupling device is a first peel off membrane removably coupled to and at least partially covering the flange front face and the sealing member.
- in pre-coupled state of the first and second aseptic coupling devices, the first membrane has a pull end part, preferably including a coupling portion with at least one rigid member, couplable with a complementary pull end part of the membrane of the second aseptic coupling device, which is a second peel off membrane.
- the membrane may include a free end at the pull end part, the free end or the pull end part including at least one snap configured to be coupled to a membrane of the other aseptic coupling device.
- the flange of the connector, for instance integrally formed in one piece with the tubular body, has an external side wall surrounding a housing or recess forming the slide.
- the slider is partly or entirely housed in the housing or recess, two parallel sliders being each present in a connection system with two connectors interlocked in the coupled state.

In some options, the external side wall has a radial opening, while a protrusion or link is provided to pass through the radial opening to allow driving the slider along the slide. The protrusion or link can be integrally made with a sliding piece forming the slider. An elastic return part may include one or two elastic tabs that provide an elastic return force orientated along a direction passing through the radial opening or a direction passing through a flange side provide with the radial opening. More generally when the slider is a locking slider, an elastic return force may be provided for preventing accidental release of the slide. The return effect is typically provided by a resilient portion (of latching means or of a spring biasing the slider) and may be involved for maintaining an engaged position of the slider with respect to the second insert (of the complementary connector in a connection state) that is
- inserted through the axial access; and
- arranged to protrude inside the housing (at the flange side opposite from the connection side).

In some options, the flange has a rectangular shape or is provided with two parallel sides. The flange may be configured:
- with an external side wall that locally includes, preferably in a corner of the flange, a side wall opening so that an abutment part of an external actuator protrudes radially inward and/or engage the slider through the side wall opening.
- the external side wall is forming an outer frame surrounding a recess, with the slider (latching means) housed in an interior volume of the outer frame, possibly of rectangular shape.

The flange may be molded from plastic material, for instance thermoplastic, and can include an intermediate annular wall interposed (with a concentrical arrangement) between an inner flange side wall and the external side wall. Such configuration may provide a compact flange with:
- a first groove of annular shape for the seal (at the front side), arranged between the intermediate annular wall and the inner flange side wall that includes an end of the cylindrical inner face of the tubular body;
- and a second groove of annular shape, with opposite orientation as compared to the first groove, defining the annular recess for receiving the slider and the insert end engaged by the slider.
The second groove may be delimited between the external side wall and the intermediate annular wall.

Embodiments of the mechanical coupling part of the connector may include one or more of the following particulars:
- the male connecting member and the female connecting member are distributed on the flange front face, so that the connector is genderless possibly with the flange being of generally rectangular section with two longer sides that are parallel to a sliding direction of the slider, which is a locking slider.
- two identical connectors are adapted to be fastened, with each insert radially offset and protruding parallel to the longitudinal axis, while each axial access of the female connecting member allows insertion along a direction parallel to the longitudinal axis.
- the insert comprises an inserting tab, possibly a flat inserting tab.
- the insert or inserting tab is shifted/offset on a first side relative to the longitudinal axis, a membrane of the aseptic attachment structure being elongated from a covering portion or covering member covering the opening (front opening) to a membrane pulling portion.
- the slider is radially movable, preferably toward the longitudinal axis of the tubular body, to engage the fastening region of the insert protruding end that protrudes beyond a transverse flange wall that surrounds the annular seal carried by the flange around the opening (central opening at the front of the connector).
- the insert fastening region may be facing radially outward when forming a groove or similar relief part, to be engaged by a part of the slider that is movable along a radial direction.
- the insert, which may have a beveled free edge or a slanted/thinned edge, may slide inside the axial access of the female connecting member until pushing radially outward the latch or slider that is movable in the housing or recess provided in the flange, peripherally around a shoulder and/or around the seal.
- a locking is obtained when a blocking bar of the slider is engaged between two reliefs of the insert or inside a groove of the insert.
- the blocking bar interconnects two sliding arms of the slider.

A position-locking system is obtained when mating the insert of the male connecting member (provided in the connector of another connecting device) with the female connecting member of the connector, combined with the slider to have an anti-return effect once the insert is fully/properly inserted. With such connectors being of the genderless type, a pair of locking sliders distributed in the two connectors can be combined with the female connecting members distributed in the two connectors, so that slider - insert engagement at a rear of a first connector flange is simultaneously obtained with a slider - insert engagement at a rear of the second connector flange, to thus obtain the coupled state and the fluid connection (after removal of the membranes). The front connection between the connectors may be allowed while hoses or tubes are connected at the respective rear parts of the connectors, which may be provided with a nozzle or barbed end suitable for fluid connection with a flexible hose/tube. Of course, the connection can be obtained only when the flanges of the connectors are mutually facing each other, with a parallel configuration allowing the respective inserts to be displaced parallel in opposite directions, until locking action is obtain to have axial immobilization of the interlocked coupling parts.

The locking may be initiated only at the end of the respective insertions, for instance with the fastening region in each insert provided at a distance of less than 8 mm, preferably less than 5 mm, from an insert free edge.

In some embodiments of the slider arranged as a locking slider mounted in the flange, the following features may be provided:
- the slider may comprise one or more pieces, for instance a single plastic piece that has a ring structure or a plate structure with a wide central opening.
- the locking slider has a generally annular shape with two opposite parallel sides and a central opening through which a rear cylindrical portion of the tubular body and an insert (typically the second insert of the male connecting member from the complementary connector mating with the connector) can pass.
- the locking slider is an unlockable locking element, provided with an outer part that is rigid.
- the locking slider may have a slot at an end that corresponds to a pushing side of the external actuator, the slot separating two flexible tabs.
- the locking slider may also comprise a bridge portion or similar inner actuation portion interconnecting the two side members forming the two parallel sides, optionally with the bridge portion suitable to be pushed, following pushing movement of the external actuator.
- the locking slider defines the retaining surface and may be in a retaining position adapted to abut against the fastening region of the insert (second insert) passing through the axial access (fastening region, which may include a retaining external annular rim of the second insert) to prevent removal of the male connecting member of the complementary connector.
- the locking slider may be flat and adapted to engage the second insert at the opposite or away from a contact area between an external actuator and a part of the locking slider located inside the housing.

In some options, a slanted surface (which can mean a beveled surface) is provided in one amongst the locking slider and an abutment part of the external actuator, the slanted surface being configured so that movement of the external actuator along the first direction causes the locking slider to be driven along an unlocking direction that is opposite to the second direction.

In some embodiments, the tubular body is provided with:
- a shoulder portion, for instance extending around the seal.
- the shoulder portion may delimit, with the external side wall, an annular recess for housing the latching means.
- the tubular end part may comprise a nozzle with or without barb(s), preferably forming a barbed end for a flexible hose or tube.

According to another aspect, embodiments provide an aseptic coupling arrangement comprising a first connecting device and a second connecting devices (identical connectors), typically interlocked using the male and female connecting members distributed in the two connectors (which are genderless connectors). With use of membranes, a connection system can be formed for fluid connection, after removal of the membranes, possibly without any rotation involved after engaging the coupling parts of these connecting devices.

Each of the first aseptic coupling device and the second aseptic coupling device is a connecting device such as above presented. In the aseptic coupling arrangement, in a pre-coupled state of the aseptic coupling arrangement (with two membranes interposed between the connector of the first aseptic coupling device and the connector of the second aseptic coupling device), the flange of the first aseptic coupling device and the flange of the second aseptic coupling device are interlocked in a flange-to-flange connection area, which is an area where the first insert and the second insert extend, the connection area being an annular connection area surrounded by:
- a first slider, which is the slider provided in the first aseptic coupling device; and
- a second slider, which is the slider provided in the second aseptic coupling device.

With such configuration, a compact connection system is obtained, while advantageously having two sliders facilitating management of the connection. The sliders are locking sliders. For the locking of the aseptic coupling arrangement, this can already be done in the pre-coupled state, so that not additional gesture is required. Since the connectors are genderless, the sliders may have a similar or identical way to be engaged. Using locking sliders, the locking may be obtained simultaneously, when achieving the axial insertion of the first and second inserts provided in the male connecting members.

In embodiments with locking sliders, the first slider and the second slider are inverted in position (thus with difference in angular position being 180°), while both extending perpendicular to the longitudinal axis (common axis for the connectors of the first and second connecting devices) to provide a locking of the pre-coupled state. The locking sliders are each of annular shape to be arranged each around one corresponding seal, while being preferably being inserted axially in the flange, at the opposite from the proximal/front face of the flange. More generally, it is understood that the locking sliders provide a locking of a final coupled state, after removal of the membranes; with the two sliding lockers having inverted positions in their respective slides.

In some embodiments involving an unlocking slider in at least one of the connecting devices, the aseptic coupling arrangement may carry a slider that selectively unlocks an engagement of a corresponding insert surrounded (partly surrounded) by this slider. The slider may be not involved for the locking action. Typically in the coupling arrangement, the first slider and the second slider, distributed in the connecting devices, have each a U-shape and are configured to:
- extend parallel, sharing same orientation, while both of the sliders are extending perpendicular to the longitudinal axis;
- each cover a corresponding flange;
wherein two pressing surfaces are distributed, preferably at a same actuation side, in the first slider and the second slider, for allowing an unclipping of the first insert and the second insert.

The flange can have a cavity with a side opening to provide a side access to the locking region. A branch or member of the slider, possibly extending straight along a transverse direction, is configured to have two positions inside the cavity. The cavity may be a peripheral cavity, not interfering with the interior space.

One of the two positions of the slider member may be an inserted position or unlatch position so that the slider member is inserted between the external side wall of the flange and the insert of the male connecting member (of the other connecting device in the connection system), in particular reaching an unlatch position in which the slider member extends radially between the flexible member of the insert (where a radial deformation is allowed) and the external side wall. In the unlatch position, the slider member may cause the flexible member to be depressed radially inward.

In some variants, a branch or member of the slider, possibly extending straight along a transverse direction, is configured to have a unlatch position inside the cavity, which is a position where the slider member extends radially between the flexible member of the insert and an end of the cylindrical wall delimiting the interior space. In such option, the slider member may cause the flexible member to be depressed radially outward, in the unlatch position (with the slider more inserted in the cavity than in the other position).

### BRIEF DESCRIPTION OF THE DRAWINGS

The figures of the drawings are now briefly described.
Fig. 1 is a perspective view of an exemplary fluid connecting device before use, with a membrane covering a connector opening, in accordance with a first non-limiting embodiment.
Fig. 2 is a longitudinal cross-sectional view showing, after removal of a membrane, structure of a connector of same type as in the embodiment of Fig. 1, this connector being genderless.
Fig. 3 is an exploded perspective view of an aseptic coupling arrangement, including the fluid connecting device of Fig. 1 and a compatible identical connecting device, each having same attachment structure with a corresponding membrane removable by a puling action.
Fig. 4 shows a coupling arrangement of Fig. 3, forming a connection system with two identical connectors mutually fastened, before removal of aseptic membranes.
Fig. 5 is a longitudinal cross-sectional view of a coupling arrangement, possibly the same as the one of Fig. 4, also showing a connection system with locking means housed in the flangles of the connectors.
Fig. 6A is a perspective view of a sliding piece according to an embodiment, which is a locker arranged to be movable along a slide formed in a flange of a genderless connector forming part of a fluid connecting device, the locker being typically involved for reaching locked state of the connection system such as illustrated in the Figs 4-5.
Fig. 6B illustrates a detail of Fig. 4, when the two lockers provide a locking of a final coupled state, after removal of the membranes; with the two lockers having inverted positions in their respective slides.
Fig. 7A is a perspective view of a sliding piece according to a variant, with an external actuator included in the sliding piece.
Fig. 7B is a cross-section view, perpendicular to a longitudinal axis, showing a detail of a coupling arrangement similar or identical to the one of Fig. 5, here with integration of the sliding piece of Fig. 7A in a slide formed by the flange of a first connector.
Fig. 8A is a perspective view of a sliding piece according to another variant, with a resilient part arranged on a same side as a rigid follower part having a corner portion suitable to convert a first pushing direction into a displacement of the sliding piece along a second direction.
Fig. 8B is a perspective view of a coupling part included in a connector that is provided with an external actuator suitable to cooperate with the sliding piece of Fig. 8A.
Fig. 8C illustrates, with a transverse cut perpendicular to a longitudinal axis of a coupling arrangement similar or identical to the one of Fig. 5, a flange providing an annular recess housing the sliding piece of Fig. 8A, with a radial opening provided in an external side wall of the flange to allow an abutment member of the external actuator to push the sliding piece, here along a direction (second direction) different from pushing direction of the external actuator.
Fig. 9A is a perspective view of a connector according to another embodiment, having a male connecting member carrying a flexible part forming the insert fastening region, with an external actuator ready to be mounted on the flange of the connector.
Fig. 9B is a detail perspective view of an embodiment for the male and female connecting members, involving a flexible member carried by the insert of the male connecting member as in the case illustrated in Fig. 9A, so that the locking and unlocking actions are a direct clipping and unclipping of the insert.
Fig. 9C is an exemplary piece forming the tubular body of the connector shown in Fig. 9A.

### MORE DETAILED DESCRIPTION

A detailed description of several embodiments of the invention is provided below, accompanied with examples and with reference to the drawings.
In the various figures, the same references are used to designate identical or similar elements.

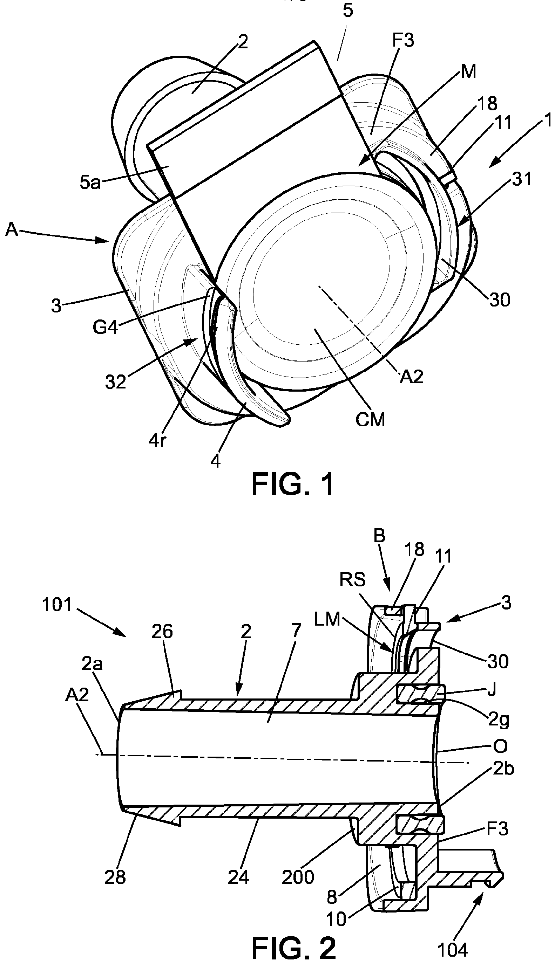

Referring to Figs 1-2, it is shown an exemplary construction of a connecting device that can be considered as sterilized, thus being treated by suitable sterilization technique, including gamma irradiation, E-beam, ethylene oxide (EtO) and/or autoclave technologies. The connecting device comprises a fluid connector 1, 101 and a membrane M forming all or part of an aseptic attachment structure 5. The connector 1, 101 of a rigid plastic material for instance, comprises a main body, which is a body 2 of tubular shape, suitable to delimit an interior space 7. The tubular body 2 can delimit a fluid passage of constant section, possibly with an inner cylindrical surface for delimiting the interior space 7. The tubular body 2 is provided with a flange 3 and may extend between a first open end 2a and a second open end 2 that is forming an opening O at a flange front face F3. The connector 1 may have a longitudinal axis A2, around which the tubular body 2 longitudinally extends. A cylindrical inner face may delimit the interior space 7, with this longitudinal axis A2 possibly being a central/symmetry axis of the tubular body 2.

As apparent in Fig. 2, the opening O may be a central opening arranged between a female connecting member 31 and a male connecting member 32 that are carried by the tubular body 2. The female connecting member 31 may be a flush mount member of the flange 3 (forming a recess in the flange 3). The members 31, 32 may, at least partly, be each included in a same single piece that forms the tubular body 2, with the flange 3 thus included as a peripheral part of the tubular body 2. The flange front face F3, forming for instance a planar face, has an axial access 30 of the female connecting member 31, while the male connecting member 32 protrudes axially from the flange front face F3.

Referring to Fig. 1, the membrane M can be removably attached to the connector 1 to (partly) cover the flange front face F3 and seal the opening O. The membrane M may have a closing member CM coupled to the front face of the tubular body 2, extending completely around the opening O and beyond this opening O at an annular attachment area. The closing member is also covering a seal J that extends annularly around the opening O. This seal J can be received in a groove 2g that opens forward. A pull portion 5a is provided so that the membrane M is removable by a pulling action exerted away from the second open end 2b. The removable membrane M prevents bio-contaminants and/or fine particles from passing through. The membrane M may be porous for gas. The membrane M may be supplemented with a stiff member, possibly perforated or provided with a recess for a coupling with a compatible (identical) stiff member provided for the other membrane M of the compatible connecting device.

The connector 1 is thus provided with an aseptic attachment structure 5 that provides a graspable portion or pull portion 5a for removal of the membrane M. Passage of the fluid conveyed through the fluidic connection using two interlocked connectors 1, 101 is allowed by having the interior spaces 7 of these connectors 1, 101 tightly communication at an interface formed with two identical seals, possibly identical seals J. The connector 1 also has a flange 3 that may extend peripherally around (all around, for instance) the cylindrical part of the tubular body 2.

### Exemplary structures of the flange in a connector

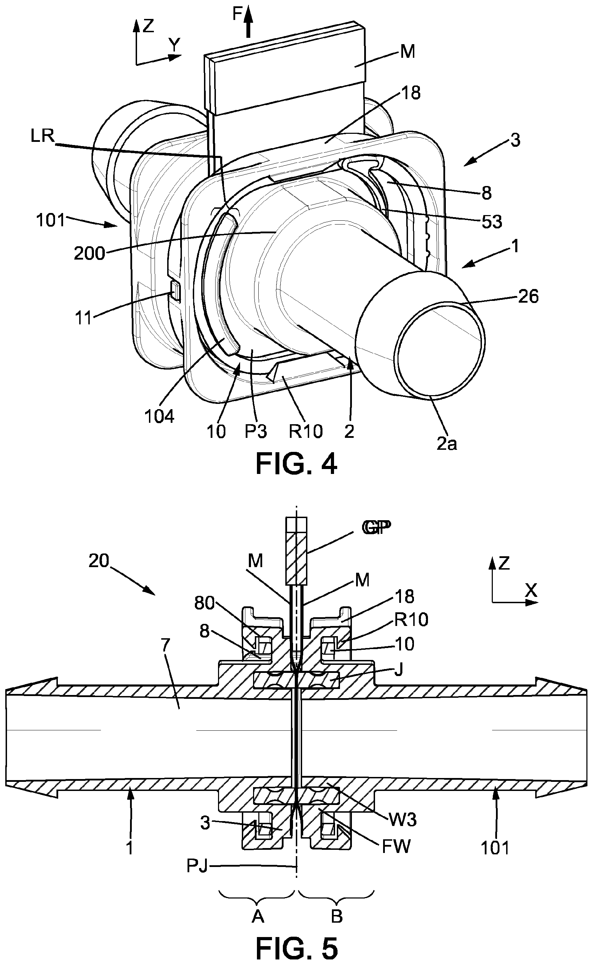

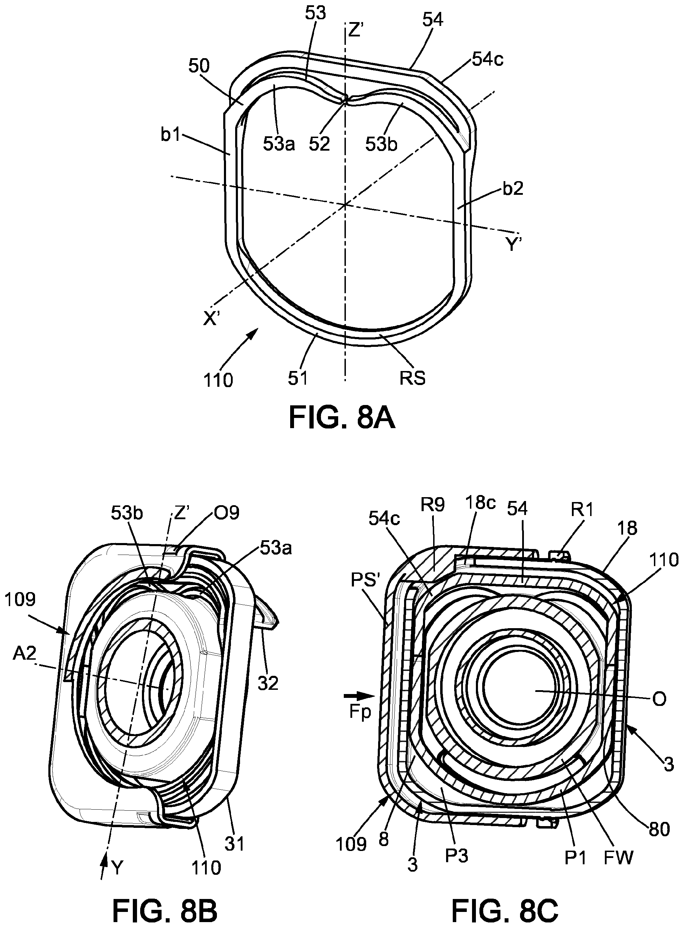

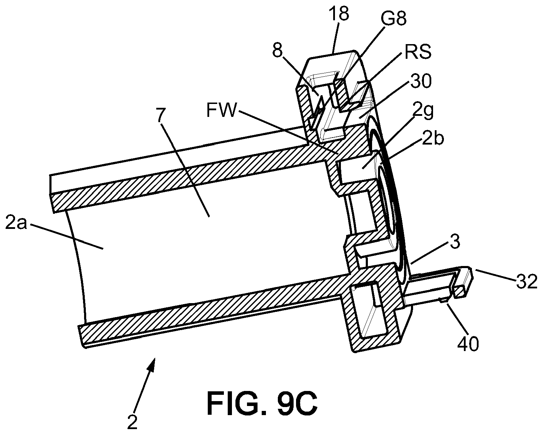

Figs 4 and 5 show an exemplary way of interconnecting two coupling parts A, B, here with connectors 1, 101 of the genderless type, without any rotation involved for instance. The flange 3, which can be molded from plastic material, can include an intermediate annular wall FW interposed (with a concentrical arrangement) between an inner flange side wall W3 and an external side wall 18, as also shown for instance in Figs 8C and 9C. Such configuration may provide a compact flange 3 with:
- a groove 2g of annular shape for the seal J (at the front side), this front groove 2g being arranged between the intermediate annular wall FW and the inner flange side wall W3 that delimits the central opening O;
- and one or more recess(es), with opposite orientation as compared to the front groove 2g, so that a slide 80, 80' is formed.

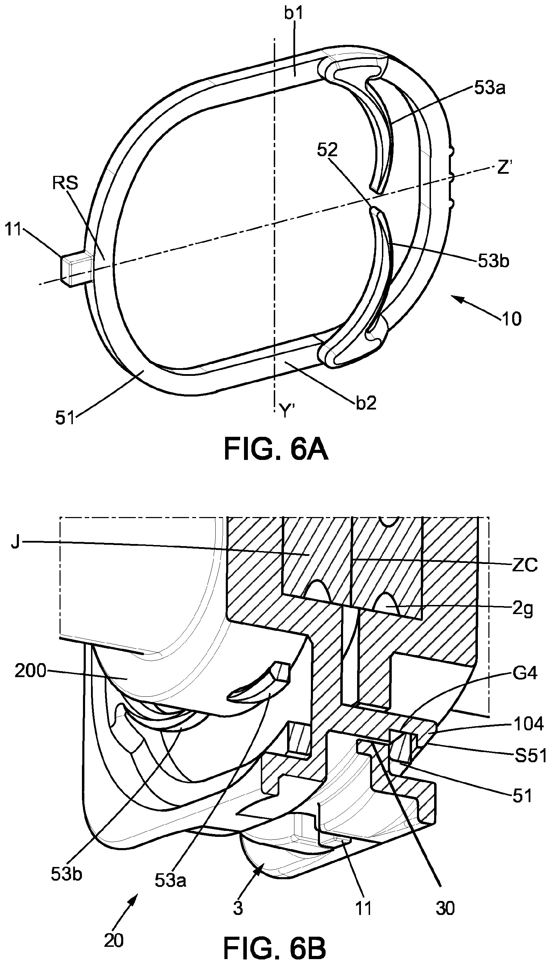

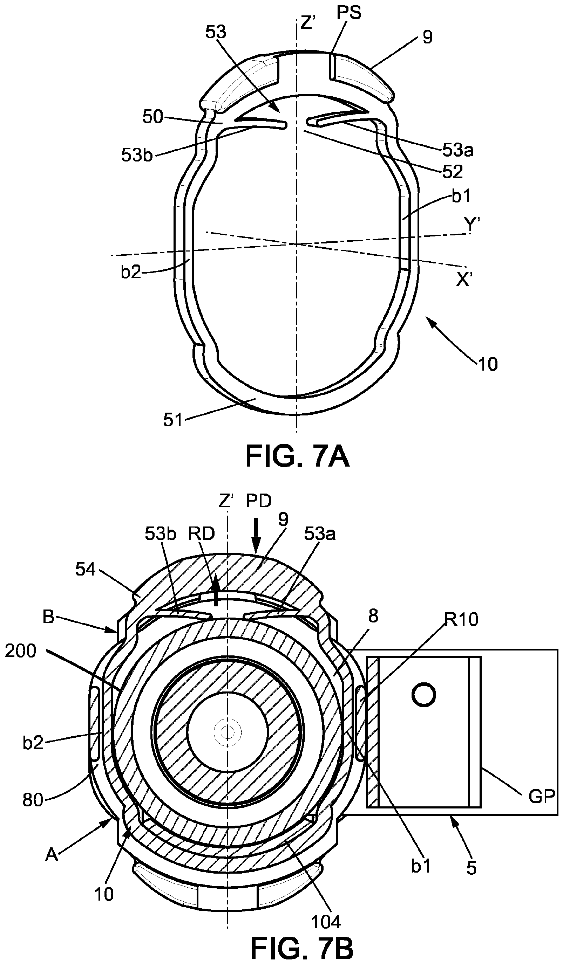

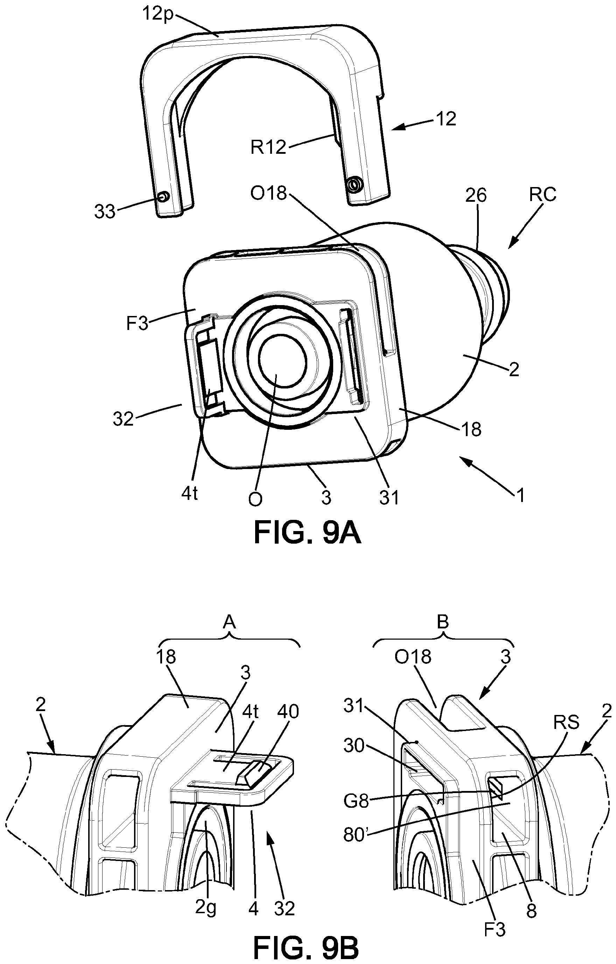

When having a slide 80 adapted for an annular sliding piece, locking of connection may be implemented, for instance using a sliding locker 10 (see Figs 6A and 7A) movable transversely relative to the longitudinal axis A2. A slide 80 may also be provided offset on a given side, possibly combined with a latch, or involving a latching part. Alternatively, a slide 80' may be provided for allowing a clipping contact or similar fastening to be released (see Figs 9A-9C), using a sliding unlocking device (such as the pushable actuating piece illustrated in Fig. 9A).

A locking region LR can be defined, typically at a rear end of the female connecting member 31. The slide 80, 80' is configured so that a movable part/slider, guided in the slide 80,80' can functionally control position of a subpart intervening for the axial retaining of the male connecting member 32. For instance, the subpart may be a flexible member 14t arranged in an insert of the male connecting member 32 or directly be included in the movable part/slider, configured as a functional element (typically a locking element) of the female connecting member 31.

More generally the flange 3 may be suitable for an efficient interlocking between two compatible connectors 1, 101, having this flange 3 and typically carrying a same kind of membrane M. As apparent in Fig. 1, the membrane M can be of low thickness, so that in pre-coupled state (before removal of membrane) it can be sandwiched/pressed directly between the two seals J distributed in the connectors 1, 101 (see Fig. 5). As shown in Figs 1-3, 6B, 8B and 9A, the flange 3 can be provided with a female connecting member 31 (offset on a given side) and a male connecting member 32 that is arranged on a different side of the flange, as apparent in the view of Fig. 1 for instance. An axial access 30 is provided at the female connecting member 31, possibly forming a through passage from the face F3 to a recess where a clipping or similar engagement with axial abutment can be obtained. This clipping or engagement is an action, at the end of the male connecting members insertion, to lock a connection between the two mating flanges 3, forming the coupling parts A, B of the connection system 100. The axial access 30 may open through the front face F3, to be adjacent to the covering member CM.

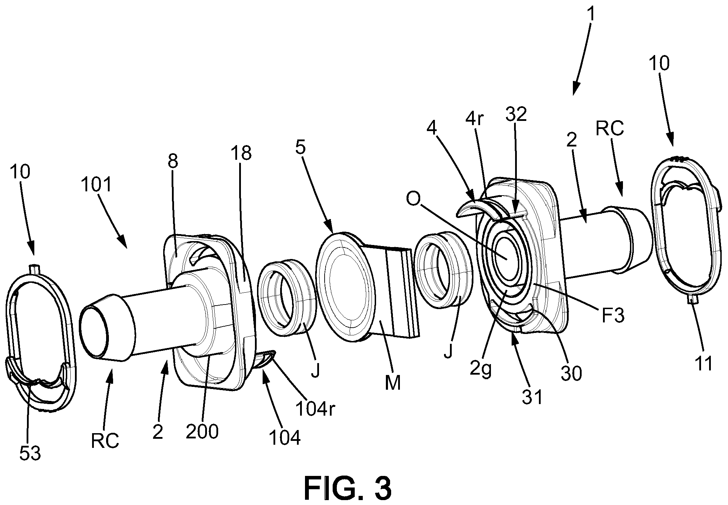

The aseptic attachment structure 5 is formed by fastening the membrane M to an annular surface of the flange 3 at the flange front face F3, around the central opening O. Such attachment surface is covered by the covering member CM (here a planar member made of the material of the membrane M) that belongs to the membrane M. The covering member CM may have a margin portion that typically contacts and/or extends around an annular seal J received in the groove 2g (front groove) of the flange 3. A complementary portion of the membrane M, possibly of rectangular shape, may extend radially outward from the covering member CM to a pull end part (at the pull portion 5a) that is accessible for the operator, i.e. provided beyond an external rim ore external side wall 18 of the flange 3.
As apparent in Figs 1 and 3, the flange front face F3 can be partly covered by the membrane M, with the female connecting member 31 and the male connecting member 32 distributed on either side relative to the membrane M. The male connecting member 32 protrudes from the flange front face F3 and may follow a curved section of the attachment surface and/or be adjacent to the covering member CM. As illustrated in Figs 2, 5 and 6B for instance, the flange 3 may have a thickness that is superior to a length (axial size) of the seals J surrounding the opening O. The (female and male) connecting members 31, 32 may be disposed close to the central opening O. Such arrangement can facilitate a manual actuation of the disconnection, with a pushing or any suitable action to be exerted from one side of the flange 3, typically using an unlocking means forming an external actuator 11.
In some embodiments, no unlocking is allowed and no actuating pushable portion is provided.

The flange 3 may be included in the tubular body 2 due to a one-piece construction (molded piece preferably), typically using plastic material such as any suitable rigid thermoplastic material. As shown in Figs 1-2, the tubular body 2 may extend around the longitudinal axis A2 that extends across the opening O, while the flange 3 may carry one or more piece(s) peripherally, around the interior space 7 and typically beyond an inner flange side wall W3 that possibly delimits the interior space 7. In the connector 1 of Fig. 1 and in the other illustrated connectors as well, the flange 3 is provided with the connecting members 31, 32 accessible at a front side, allowing a front connecting with another connector 101. Typically, each of these members 31, 32 is included in a single piece forming the flange 3 (and possibly also delimiting the interior space 7, by forming all or part of the tubular body 2).

The female connecting member 31 and the male connecting member 32 may be distributed on two opposite peripheral areas of the flange 3 that are not covered by the membrane M. More generally, a genderless aseptic connecting device is obtained, which allows mating engagement of the connector 1 (as shown in Fig. 1) with an identical complementary connector 101 (such as shown in Fig. 2) to have the fluid connection, provided that each membrane M interposed between the interior spaces 7 (fluid pathways) has been removed. Possibly before such removal (with each membrane still present, typically), a clipping interface forming the female member 31 can allow locking a pre-coupled state of the connectors 1 and 101. Figs 4 and 5 illustrates an exemplary connection system 100 in this pre-coupled state, obtained after clipping two inserting tabs 4, 104 or similar inserts forming the male connecting members 32.

### Interface for connection between two connectors in pre-coupled state

In a connection system 100 combining two connectors 1, 101, two coupling parts A, B of identical or similar compatible structure may be used. The locking regions LR (Fig. 4) may be diametrically opposed relative to X-direction. In illustrated embodiments, the two couplings parts A, B are identical and provided in genderless connectors 1, 101. The coupling is obtained without rotation along the X-direction as shown in Fig. 5, this X direction being here parallel to the longitudinal axis A2 of each connector 1, 101. Each flange 3 is carrying a membrane M extending along a direction, here Z-direction, which is perpendicular to the longitudinal axis A2 and also substantially perpendicular to Y-direction (Fig. 4) along which a slider is movably mounted on the flange 3. In the illustrated embodiments, Y-direction may be considered as a left-right direction, while Z-direction can form a substantially vertical direction.

As illustrated in Figs 4-5, a coupling arrangement 20 is obtained when reaching the pre-coupled state with the locking established (each retaining surface RS being engaged in the housing 8). The inserting tabs 4, 104 are thus forming a first insert of the connector 1 (Fig. 1) and a second insert of the connector 101 (Fig. 2), which are here identical connectors so that the interior spaces 7 are joined and delimited by a fully cylindrical inner face of the coupling arrangement 20.

The pair of inserts, forming inserting tabs 4, 104 or similar axial protruding parts in the coupling parts A, B, may each have an engagement rim, due to presence of a groove G4 or a radial protruding lug. Each of the inserts provided at the male connecting members 32 extend inside and through the axial access 30 provided in the corresponding female connecting member 31. More generally for a given connector 1, 101 to be used in such connection system 100, at least one female connection member 31 may be involved for an axial retaining effect, thus maintaining a contact between the seals J of annular shape, which may be an axial contact when having connectors 1, 101 of identical or similar structure.
When reaching full insertion of the inserts of the male connecting members 32, a locking can be obtained using latching means LM (Fig. 2) that can be arranged at the rear of each flange 3. Then, after such mechanical connection, the membranes M can be removed to obtain a fluid communication.

The mechanical connection uses such female connecting member(s) 31 that is provided with a retaining surface RS, this connection involving a corresponding insert provided by the male connecting member that is engaged and locked in properly inserted position. The connection may correspond to a removable or permanent fixation.
When being a removable connection, a quick disconnection is available, typically requiring unlocking the fixation means. As apparent from Figs 7B, 8B and 9B, an external actuating part 9, 109, 12, typically acting as a push button, can be provided for each connector 1, 101.

In the connection system 100, each connector 1, 101 may be genderless while also carrying a movable part , forming a slider 10, 110, 12, that can be inserted in a transverse recess/housing 8 (forming a slide 80 as reflected in Figs 5, 7B, 8C) or through a radial opening O18 (Figs 9A-9B) to allow disengaging a retaining rim/surface or interfering portion 51 that cooperates with the end/insert of the male connecting member 32. When having a locking slider 10, 110, the interfering portion 51 may be part of the locking slider 10, 110. In other words, the latching means LM can be carried by the flange 3 and be constructed as a sliding piece.
For the type of connector 1, 101 shown in Figs 4 and 6B, each flange 3 can delimit a slide 80 and may be provided with a slider, possibly under the form of a sliding piece, arranged along the slide 80 in a housing 8 (typically delimited by the external side wall 18 of the flange 3). The structure of the flange 3 may be provided with a slide 80, 80' adapted to allow an adjustment in position of one of the interlocked portions amongst the insert and the retaining surface RS provided by the flange 3 at the insertion passage belonging to the female connecting member 31.

It is understood the slider 10, 110, 12 can be arranged around and spaced from the interior space, or offset relative to the interior space 7, thus having no seal member/portion carried by the slider 10, 110, 12.

In some embodiments, a curved profile is used for an inserting tab 4 (Fig. 1) of the male connecting member 32 and a curved shape can be used as well for the axial access 30 delimited by the female connecting member 31. The size and shape of each profile, for the inserting tab 4 and the axial access 30 may be matching so that the connector 1 can cooperate with an identical connector 101 having same male and female connecting members 31, 32. Figs 5, 6B show all or part of a connection system 100 with the two connectors 1, 101 coupled at coupling parts A, B that are of identical structure. Each coupling part A or B may include a flange 3 as above disclosed (using a slider 10, 110 directly retaining the insert), with the aseptic attachment structure 5. In such options, the retaining surface RS may extend at a side of an annular sliding piece or similar slider, possibly mounted axially at a rear face of the flange 3 and retained by retaining tabs R10 adjacent to the slide 80.

Referring to embodiments of Figs 1, 6B, 7A-7B and 8A-8C, with a locking action involving a slider 10, 110 that is distinct from the flange 3, it is understood that an exemplary inserting tab 104 provided in the coupling part B of a connector 101 can automatically be engaged and locked by the slider 10, 110. An elastic return part 53 can be included in the slider 10, 110, to allow the interfering portion 51 to be provisionally offset before returning to its initial position be housed in the groove G4 of the inserting tab 104. Of course, any suitable fastening region 4r, 104r may be provided for each of the inserts, possibly under the form of inserting tabs 4, 104.
As apparent in Fig. 6B for instance, the inserting tab 104 is inserted to protrude at the rear side of the flange 3 of the coupling part A provided in the connector 1, beyond a rear end of the axial access 30. The inserting tab 104 can transversely displace the slider 10, 110 at the interfering portion 51 when being inserted between the slider 10, 110 and the tubular body 2. At the end of the insertion, as illustrated in Fig. 4, the inserting tab 104 protrudes from a bottom wall P3 (that is perpendicular to the axis A2) and the interfering portion 51 is biased radially inward by biasing means to retain the inserting tab 104 at the fastening region 104r (Fig. 3) of the insert/inserting tab 104.

In the connection system 10, the fastening region 104r and a fastening region 4 (Fig. 3) of the insert 4 can be engaged to lock the flange-to-flange connection. A transverse guillotine system, compact along the axial direction (along the X-direction or axis A2), may allow the interfering portion 51 to be received in the groove G4 formed at each insert (insert tab 4 or 104), possibly with a return effect allowed by an elastic return part 53 as described later. When having a transverse guillotine system, possibly using a locking slider 10, 110 of annular shape to provide the interfering portion 51, external actuation for uncoupling the system 100 may be a simple pushing action, exerted peripherally on one side of the flange 3. This allows an easy disconnection to allow axially separating the flanges 3, typically with a pulling of the connectors 1, 101 in opposite directions, while the pushing is exerted to release the respective fastening regions 4r, 104r.
Other variants may be implemented, possibly using an external sliding part that is rigid for interacting with a flexible member 4t of the inserting tab 4, as in the case illustrated in Figs 9A-9C.

Referring to Figs 3, 4, 5 and 7B, the connecting devices (with the connector 1 and with the connector 101, respectively) have each an offset radial portion included in or parallel to the membrane M, belonging to the aseptic attachment structure 5. This radial portion is possibly radially spaced from the tubular body 2. In assembled state of the two connectors 1, 101 (with locked position), the radial portions, forming the respective pull portions 5a, may form a common graspable portion GP (Fig. 7B) to control/facilitate removal of the membranes M. The hoses/pipes or devices may be connected to the connection system 100 obtained with such graspable portion GP extending radially in the pre-coupled state of the connection system 100.

### Examples of position-locking system to prevent accidental disconnection

Referring to Figs 2, 3A and 4-5, the coupling arrangement suitable to form the connection system 100 (with the connector 1 and with the connector 101, respectively) may be provided with a pair of locking sliders 10, 110. The connector 1 as shown in Fig. 2 include the locking slider 10 such as shown in Fig. 6A, which is of annular shape and provided with an interfering portion 51 on a given side of the slider 10. Accidental disconnection of the male connecting members 32 is prevented by having a clipping or snapping action enabled by each of the sliders 10. No external actuator is provided in embodiments of Figs 1-2 and 6A-6B, only a small protruding lug or tab 11 being provided as part of the slider 10 that extends through the external side wall 18 of the flange 3. Unless adding a pullable insert at such tab 11 and/or enabling selectively pulling this tab 11, possibly with use of a tool, the slider 10 cannot be externally actuated. In this illustrated embodiment, no pullable insert or anchoring feature is present and no sliding is allowed after the clipping (clipping to axially retain the inserting tab 104).
In some options, the tab 11 is simply a guiding part for the mounting of the slider 10. Possibly, the external side wall 18 may entirely surround the slider 10 that is performing a permanent clipping, without any possibility to unlock the connection system 100.

More generally, the locking slider 10, 110 may be formed in a plate or similar single piece. A return part 53, included in this piece, may exert a return effect along direction RD. This prevents accidental disconnection. When having an external push-button/actuator 9 or external actuator 109, as illustrated in Fig. 7B and 8B, a pushing along a pushing direction PD for the pushing can be actuated from a side of the connector 1, to initiate/allow disconnection. This pushing increases the compression or flexing of the return part 53, so that stopping the pushing immediately causes the slider 10, 110 to be moved in the direction RD (return direction) opposite to the pushing direction PD. The actuator 9, 109 may be assembled with or included in a part of the slider that includes the sliding portion(s), typically two sliding arms b1, b2.

Stability of the connection system 100 is here obtained by insertion guiding means formed at the axial accesses and by latching means LM, under form of a slider 10, 110, that are each stably maintained in the housing 8, in order to avoid accidental disconnection due to accidental removal of the male connecting members 31.
Referring to Figs 6A, 7A and 8A, it can be seen the slider 10, 110 may have an annular shape using four narrow sides (of low thickness), so that the latching means LM may be formed with optimized use of plastic material. Two parallel arms b1, b2 allow the sliding. The locking slider 10, 110 may optionally ensure a combined retaining action and sliding action, around the tubular body 2.

The locking slider 10 may be clipped, using lugs or outer reliefs R10 (Figs 4, 5, 7B) that locally protrude inwardly from the external side wall 18. For instance, the reliefs R10 are arranged/distributed at opposite sides along the Z-direction and configured to allow, after mounting, a further displacement of the push-button or external actuator 9, so that a sliding is obtained in response to pushing actuation on a proper pushing surface PS at an accessible end, which is either an end directly provided on the locking slider 10, or provided on a separate external actuator/button 9. The tabs or retaining reliefs R10 may form means for definitively retaining the locking slider 10, 110.

### Options with a single sliding piece forming the slider and an actuating portion

In some options, as shown in Figs 7A-7B, an external actuator 9 is included in the piece forming the locking slider 10: the pushing direction PD is the same as the sliding direction, for the unlocking operation, so that the locking slider 10 can be unlocked, thus releasing the insert.

Referring to Figs 7A-7B, the actuator 9 can be substantially as narrow as the remainder of the slider 10. The slider 10 is here provided with the actuator 9 at the opposite from the interfering portion 51, allowing a pushing along Z'-axis direction. With such configuration, using retaining tabs or reliefs R10, the external side wall 18 may simply be formed by two opposite wall portions to form the slide 80, so that the actuator 9 can be movable at a first side called pushing side. The opposite side (away from the pushing side) may also be an open side, the slider 10 being stably received in the slide 80 due to the flexible tabs 53, 53b that can be symmetrically arranged to stabilize position of the slider along Z'-direction (Fig. 7B) that is matching with left-right direction (Y direction as shown in Fig. 4) when the membranes M are disposed vertically.

### Options for unlocking the slider by a pushing actuation not parallel to the slide

While a slider 10 carrying a push-button 9, preferably of one-piece construction, has been illustrated in embodiments of Figs 7A-7B, variants are also proposed to have an external actuator 109 that is movable, relative to the flange 3, to drive the slider 110 and control unlocking of a connection system 100 similar to the system of the type illustrated in Fig. 5. Fig. 8B illustrates a case where the external actuator 109 can be pushed (arrow Fp) along a pushing direction that is parallel to the extension direction of the membrane M of the connecting device. More generally, any suitable external actuator may serve to drive the slider 110 from outside the flange 3, with a driving involving a change of direction, possibly using a cam surface.

Referring to Figs 8A-8C, it is understood that the slider 110 (of annular shape or of any suitable shape to form latching means LM) consists in a locking slider 110 that is sliding along a direction perpendicular to the pushing direction. The manual pushing (arrow Fp) may be exerted at an external actuator formed as an external part or external actuator 109, covering the flange 3 on at least one side. Referring to Fig. 8C, it can be seen a housing 8 may be defined around the flange intermediate wall (that surrounds the seal J), radially beyond the groove 2g for the seal J, so that the slider 110 may be carried (preferably inside the housing 8) by the flange 3. that also carries the external actuator 109. The external actuator may be slidably mounted on the flange 3. While the external actuator 109, provided with a pressing/pushing surface PS', has here a structure of a push button with two outer arms forming two parallel sliding sides for having a mobility on the flange 3, other constructions may be used, possibly using a hinged actuator or any suitable structure with a movable part.

The external actuator 9, 109 may be a push-button, may be C-shaped, U-shaped and/or configured to cover three distinct sides of the flange 3, while being slidably mounted along a given direction, which may direction of the axis Z when forming an external actuator 109 (see Figs 8A-8C). When the external actuator 109 comprises two sliding outer arms, the female connecting member 31 and the male connecting member 32 can extend each in an intermediate position between:
- the intermediate wall FW that surrounds the seal J,
- and one of the sliding outer arms.
Openings O9 may cooperate with outer reliefs R1 (Fig. 8C) of the external side wall 18, with abutment contact of the reliefs R1 against rim(s) of the opening O9, to prevent separation of the external actuator 109.

Regarding a non-limiting exemplary structure of the locking slider 110, it is here proposed a one-piece structure with integration of a specific corner region pushable by an abutment member R9 of the external actuator 109, which is an abutment member arranged in a junction between one of the tow outer sliding arms and the covering portion forming the pushing surface PS'. The corner portion 54c in the slider 110 can be selectively pushed by the abutment member R9 so that the pushing is converted into a perpendicular sliding of the locking slider 110.

Whatever the way the structure of bridge portion 54 is designed, it can be in contact with an abutment member R9 of the external actuator 109, which is arranged at an end of the locking slider 110. The bridge portion 54 can optionally be elongated to extend along a side of the external side wall 18 that is open at a radial opening. Fig. 10B shows an exemplary circumference of the external side wall 18, with the radial opening 18c allowing the abutment member R9 to engage the locking slider 110.

As in the version of Fig. 6A or 7A, the locking slider 110 may have two sliding arms b1, b2 distributed symmetrically, relative to the axis Z' (the two straight sliding sides b1, b2 being typically at equal distance from such axis Z'). This axis Z' can match with Y-direction of the connector 1, 101 in mounted state of the locking slider 10. The slide 80 allows sliding displacement of the locking slider 110 along direction of the axis Z'. The locking slider 110 may have a slot 52 at an end that corresponds to a pushing side. The slot 52 is separating two flexible tabs 53a, 53b that may form the return part 53, which is possibly axially offset relative to a bridge portion 54 provided with the corner portion 54c. The bride portion 54 is not accessibly from outside the flange but can be pushed via the abutment member R9.

The flexible tabs 53a, 53b, extend away from a longitudinal axis X' of the locking slider 110 and can resiliently engage an outer region of the tubular body 2, as shown in Figs 8B-8C, thus biasing the locking slider 110 toward a locking position, in which the interfering portion 51 is engaging an inserting tab 4 or 104 to prevent flange-to-flange disconnection. The external actuator 109 covers the bridge portion 54 and a side of the flange that covers the bride portion 54. A junction 50 can be provided between the bridge portion 54 and the arms b1, b2, to have the locking slider 10 formed as a closed loop.

The flexible tabs 53a, 53b may extend each from an arm end (at junction 50) to a free end delimiting a side of the slot 52. In advantageous options, the two flexible tabs 53a, 53b can be arranged symmetrically with respect to the X'Z' plane. Such configuration may be also adopted (entirely or partly) for the other locking sliders 10.

In addition, the flexible tabs 53a, 53b are arranged (along Z'-direction) under the bridge portion 54. Typically, in mounted state, they are interposed between the bridge portion 54 and a continuously curved surface of the intermediate wall FW. In addition, one will note that each of the flexible tabs 53a, 53b has a curvature oriented away from a central region of the bridge portion 54 (curving downwards in non-limiting embodiment of Fig. 8A). In addition, each of the flexible tabs 53a, 53b optionally has a cross-section or thickness which decreases from its root to its free end (where the slot 52 is delimited).

Referring to Figs 6B, 7B, 8B, the elastic return part 53 may be configured to be in radial contact against the shoulder part 200 or similar portion of the tubular body 2. For the connection, when an inserting tab 4 or 104 engages on the interfering portion 51 provided in the housing 8 (annular housing), starting to axially slide on optionally beveled surface forming a front side of the interfering portion 51, a clipping is obtained once the interfering portion 51 reaches the groove G4 as illustrated in Fig. 1.

It is understood the slider 110 cannot be disengaged with respect to the insert (inserting tab, typically), unless being displaced by the suitable sliding part or abutment member R9 (Fig. 8C), which is a part carried or integral with the external actuator/button 109, which is compatible with use of genderless coupling parts A, B. While the push-button is here illustrated as being protruding outside with respect to the flange, a push-button recessed inside the flange may also be provided with a pression surface PS that remains accessible from outside the flange.

The pushing surface PS' thus may be provided to have a pushing direction (see arrow Fp) that is parallel to direction of pulling the membrane M. Having such orientation for the external actuator 109 can be of interest, for instance if a simultaneous pushing is wanted for a simple disconnection. A fastening of two adjacent external actuators can be possible, with use of same direction, provided the actuators can be indifferently mounted in the two orientations of the Z-direction.

### Option(s) without any transverse locking slider between the flange and the insert

While a locking slider 10, 110 may be used in many embodiments, the connector 1 can alternatively allow a locking action when the mechanical connection between the connectors 1, 101 is completed, only by interaction between the flanges 3 provided in these connectors 1, 101. In such case, a flexible member 4t may be provided in the insert forming all or part of the male connecting member 32. Referring to Fig. 9A, a slot arrangement, a U-shaped slot or any suitable separation or reduction of thickness may be provided in the male connecting member 32 to separate the flexible member 4t from a remainder part of the insert, which may be an inserting tab 4, 104.
More generally, the slider that is movably mounted on the flange 3 can be an unlocking slider, in embodiments provided without any annular ring part(s) to lock the coupled state. The locking may involve a retaining action by abutment engagement between the insert (each insert typically) at the flexible member 4t and the retaining surface RS provided in the female connecting member 31.

The flexible member 4t may be provided with a radial protruding lug 40. The radial protruding lug forms a retaining rim to obtain an axial abutment, when engaged against a retaining surface RD surface of the flange (or surface of a latching means LM carried by the flange 3), when the lug 40 extends beyond the axial access 30 in the female connecting member 31. For the mechanical coupling, each inserting tab 4, 104 may have a free end engaged/received in a groove G8 (Figs 9B-9C) arranged in the housing 8, while the flexible member 4t (possibly separated from the free end by a slot or thinning portion) is engaged at a rear of the retaining surface RS, here included in a rim portion of the flange 3. Like the case of a locking slider 10, 110:
- position of the retaining surface RS may be intermediate between the external side wall 18 and the intermediate wall FW,
- and/or a sliding displacement of the external actuator forming the slider 12 allows disengagement between the flexible member 4t and the retaining surface RS, for instance by having the abutment member sliding in a slide 80' of the housing 8 to push the lug 40 or similar retaining relief of the flexible member 4t, preferably with a centripetal pushing, thus causing a flexing of the flexible member 4t that is no more facing the retaining surface RS.

Referring to non-limiting option of Fig. 9B, the retaining surface RS can be directly included in a single piece forming the flange 3 (and possibly also forming the tubular body 2), provided the insert has a flexible member 4t with a radial relief suitable to be engaged on a flange rim forming the retaining surface RS. With such arrangement, the slider 12 can be configured, as shown in Fig. 9A, as an external part actuatable to unlock engagement of the flexible member 4t of the insert with the retaining surface RS.

Also in such embodiments, gesture is simple as a sliding action for the slider 12 may cause the flexible member 4t to be radially moved and disengaged from the retaining surface RS. Starting from a first position, the slider 12 can be directly pushable to selectively engage the radial protrusion of the flexible member 4t so that the flexible member is in a flexed state (flexed radially inward), in a second position (pushed position) of the slider 12.

While this example is illustrated with a flange 3 that is laterally slotted, other configuration may be provided for having the slider 12 interacting with a rear opening of the axial access 30. The slider 12 can be configured to push or displace the at least one relief of the second insert, by a contacting portion R12, possibly carried by a sliding outer arm or similar sliding part. The slide 80' may be perpendicular to the axial access 30 and adjacent to this axial access 30. The slider 12 can move in the slide 80' to move the radial relief (or any suitable relief(s) of the flexible member 4t) out of the slide 80'. When reaching such pushed/actuated position, the slider 12 changes configuration of the locking region LR, so that the locking configuration is unlockable. Here, unlocking is performed by an unclipping action since flexed state/position of the flexible member 4t allows the second insert end to be moved back in the axial access 30.

The sliders 12 can be used with a coupling arrangement 20 similar to the one shown in Fig. 5, except for configuration of the slide 80' in the flange 3 and retaining region (in the flange 9) for mounting each slider 12. The sliders 12 may have each a U-shape compatible with extension of the membrane M (with pull portion 5a extending at the uncovered side of the flange, for instance). The sliders 12 of the coupling arrangement 20, each extending perpendicular to the longitudinal axis A2 (X-direction), can be configured to:
- extend parallel, sharing same orientation;
- each cover a corresponding flange 3;
- have a pressing surface 12p.
The pressing surfaces 12p may be distributed in the sliders 12 at a same actuation side for allowing an unlocking required for the unclipping of the first insert and the second insert.

Optionally, using the pins and pin receivers or similar synchronizing means 33 (here forming a linking part), at the front of the sliders 12, unlocking and disconnection of the fluid connection is allowed by simultaneous displacement of such sliders configured as external actuators for the unlocking. While the connectors 1, 101 are genderless with identical structure, orientation of the sliders 12 (each of identical structure) may be different for obtaining two compatible connecting devices.

### Method of joining two connectors

Referring to Figs 1-2 and Fig.5, the method can involve fluid coupling components A and B that are identical, each provided in a connector 1, 101 of similar or identical structure, here genderless. The opening O may be a central opening, so that it may provide an axial passage, through the flange 3 and extended (rearwardly) by the tubular body 2. This axial passage is rotationally symmetrical about the longitudinal axis X, allowing passage of the fluid conveyed through the fluidic connection.

This method firstly involves forming a coupling arrangement 20, comprising a first aseptic coupling device and a second aseptic coupling device, with the connectors 1, 101 interlocked at the coupling parts A, B. The following steps may be performed to achieve sterile joining of the two connectors 1, 101, although not necessarily in the order described:
- sterilizing the connectors 1,101 and the aseptic attachment structure 5, with each of the connecting devices being axially covered at least at the flange front face F6 by a corresponding membrane M of the attachment structure 5;
- assembling each connector 1, 101 with a tubing, a hose or the like, possibly using a barbed nozzle (with barb 26), or establishing a suitable fluid connection with an equipment (in some options a rear end of the connector 1, 101 may be also provided with a genderless connector);
- aligning the inserting tabs 4, 104 or similar inserts with facing axial accesses, by adjusting angular position of the flanges 3 of the connectors 1, 101;
- displace the sterilized connectors axially (X-direction), to have a flange-to-flange connection with each insert of the male connecting members 32 fully inserted through the corresponding axial access 30 of the female connecting member 31, so that the covering members CM of membranes M are adjacent with each other;
- obtaining the pre-coupled state of the connection system 100, thus obtaining the coupling arrangement 20, such as illustrated in Figs 4-5 for instance, i.e. with the membranes M extending along a junction plane PJ;
- removing, simultaneously, the protective membranes M from the connectors 1, 101, by exerting a pulling force on a common pull portion (typically a graspable portion GP) to immediately obtain axial contact between the seals J (O-rings), without any relative rotation between the flanges 3.

In the pre-coupled state, two membranes M are extending parallel and in mutual contact, before a combined removal of these membranes M. While hose nozzle with at least one barb 36 or similar connecting male member has been illustrated at the rear end of each connector 1, 101 or at a rear of a fitting RC, it is understood than any other connecting part may be used, possibly with the connector directly welded on a pouch, a bioreactor or any device used in a circuit where a biopharmaceutical fluid can flow.
From a control point of view for a "single use" system, each connector 1, 101 in the connecting device is compliant with starting a connection, which is aseptic. The genderless structure allows using the connecting devices (with connector 1, 101), with formation of the coupling arrangement 20, at several interfaces of a biopharmaceutical circuit.

Referring to Figs 1-2, 5 and 6B, the width of each inserting tab 4, 104 may be superior or equal to depth of the groove 2g. At least one bevelling may be provided at a free end of the inserting tab 4, 104, to help in having a guided insertion, simultaneously, of the inserting tabs 4, 104, in opposite directions. The locking is also performed simultaneously and automatically, using for instance two locking sliders 10, 110 interfering at an area (locking area) of the recess/housing 8. At this locking area, the axial access 30 opens for the passage of a fastening region 4r, 104r orientated radially outward on each inserting tab end.

For having such locking action, the locking slider 10, 110 is shown as an exemplary piece forming the latching means LM, using an annular shape fitting with the annular shape of the housing 8, possibly using retaining tabs R10 for only allowing a sliding of the locking slider 10, 110. Of course, other structures may form the slider, for instance using a recess arranged only in a given angular sector in the flange 3, where the inserting tab or insert to be engaged extends. This angular sector may correspond to the side where a pushing is exerted on the external actuator 9, 109, when unlocking of the connection is allowed.

In the connection system 100, the two connectors 1, 101 can be coupled so that the external actuators 9, 109 are both pushable, possibly from different directions, possibly opposite transverse directions as reflected by Fig. 7B for instance.

Then, after mating the coupling parts A, B, using the aseptic attachment structure 5, an aseptic connection can be made between the two connectors 1, 101. After removal of the membranes M, with the seals J in mutual contact, there is no requirement for the user to rotate or actuate any driving part to further displace or deform the flanges 3 and the seals S. In some variants, at least one of the connectors 1, 101 used in the fluid connection may be arranged with a plug, for instance with a plug that is spring biased or driven by a bayonet connection.

It is understood that, once the membrane(s) M is no more present due to a connection, the locking action by the two sliders 10, 110 can be obtained to enable an axial pressure to be exerted on each O-ring or seal J, thus ensuring a sealing function around the central passage defined at the openings O. The sealing elements or seals J may have an excess in length to ensure an axial pressure is exerted in such annular contact area, forming an annular junction (Fig. 6B).

The insertion movement can be completed when having the locking slider 10, 110 engaged in the groove G4 that forms all or part of the fastening region 4r, 104r. Figs 6B shows that the inserting tab 104 (idem for inserting tab 4), passing through the axial access 30, can thus be interposed radially between the intermediate wall FW (possibly forming the circumferential outer surface of a shoulder part 200) and the interfering portion 51 of the locking slider 10. The elastic-return part 53 prevents radial displacement of the interfering portion 51, unless the locking slider 10, 110 is displaced along the slide 80 by a pushing action exerted at the pushing surface PS, PS'.

The bottom wall P3, the external side wall 18 and the intermediate wall FW can form the housing 8, made integral with the tubular body 2. Referring to Figs 4-5, the external wall 18 can optionally provide two parallel guide portions and two straight side portions are included in the projection 200, forming opposite parallel sides, all extending along a same transverse direction Y.
Referring to Fig. 6A or 7A symmetry axis Z' of the locking slider 10, may optionally define a length direction of the locking slider 10.

Use of slides 80, 80', distributed in the connectors 1, 101 of the connection system 100 such as shown in Fig. 6B, allow forming relatively flat flanges 3 since each slide extends in a transverse plane YZ perpendicular to the longitudinal axis X. Moreover, when having a locking slider 10, 110, mounting of the locking slider 10, 110 by the rear face of the flange 3 is of interest to have an efficient connection with appropriate sealing contacts and proper aseptic attachment structure 5. The locking slider 10, 110, possibly formed as a molded plastic part comprising a wide-open locking plate, may be a flat piece (compact piece along longitudinal axis X), with a central opening through which a part of the intermediate wall FW of the flange can pass.

The tubular body 2 can be formed of plastic materials biocompatible with biopharmaceutical substances, for example polypropylene, polyethylene, polycarbonate, polysulfone, polyvinylidene chloride, polyethylenimine. The open end 2a can optionally be provided in a suitable fitting RC of a tubular body end 28, for instance molded with the flange and having at least one hose barb(s). It can also be provided in a clippable interface adapted to permanently clip the connector 1, 101 to a compatible component (assembled component). Alternatively, the open end 2a can be arranged directly in a flange structure compatible or identical to the structure of flange 3. It is understood the connector 1 with the coupling part A formed at the flange 3 (possibly including a ring forming the locking slider 10, 110) can be adapted to be assembled to any component used for fluid transfer, possibly by including a coupling part B at the rear end provided with the open end 2a.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A connecting device for fluid connection, having a connector (1) that is genderless, the connecting device comprising:
- a tubular body (2) extending around a longitudinal axis (A2) of the connector and delimiting an interior space (7), the tubular body having an opening (O) at a front of the tubular body (2) where the interior space (7) axially opens;
- a flange (3) formed peripherally on the tubular body (2) around the interior space (7), the flange (3) comprising a female connecting member (31) that is provided with a locking region and an axial access (30), the axial access (30) preferably opening at the front of the tubular body (2), and a male connecting member (32) protruding axially from a flange front face (F3) to provide a first insert having a fastening region (4r);
- a sealing element (J) providing an annular contact surface at the flange front face (F3), radially interposed between the opening (O) on the one hand, and the axial access (30) and the male connecting member (32) on the other hand; and
- a slider (10; 110) mounted on the flange (3) to be movable relative to the flange, along a direction (Y; Z) transverse to the longitudinal axis (A2);
wherein the slider (10; 110) is arranged as a functional part of the female connecting member (31), the slider (10; 110) being adapted to modify the locking region provided with a retaining surface (RS), the axial access (30) allowing insertion of a second insert, of same structure as the first insert and thus also having a fastening region (104r), the second insert being part of an identical complementary connector (101) genderless for the fluid connection and being sized and shaped for engagement of the retaining surface (RS) with the fastening region (104r) of the second insert,
the flange (3) forming a slide (80) for the slider (10; 110) that is a locking slider of the female connecting member (31).

2. The connecting device according to claim 1, comprising:
- an aseptic attachment structure (5) including the flange front face (F3) and a membrane (M) that is removably attached to the connector (1) at the flange front face (F3) to seal the opening (O).

3. The connecting device according to claim 1 or 2, wherein the retaining surface (RS) is configured to axially retain the second insert at the fastening region (104r) thereof in a locking configuration of the fluid connection, the second insert being formed in a complementary connector (101) that is suitable to be coupled to said connector (1) for enabling the fluid connection,
the locking configuration being obtained when the second insert passing through the axial access (30) is inserted in the female connecting member (31) to have a second insert end extending axially beyond the retaining surface (RS),
wherein the slider (10; 110) has two parallel sliding arms (b1, b2) distributed on two sides in the flange.

4. The connecting device according to claim 2 and 3, wherein the membrane (M) is configured to be pulled radially along a pulling direction,
and wherein the two parallel sliding arms (b1, b2) are configured to slide along a direction that is perpendicular to the pulling direction.

5. The connecting device according to claim 2 and 3, wherein the membrane (M) is configured to be pulled radially along a pulling direction,
and wherein the two parallel sliding arms (b1, b2) are configured to slide along a direction that is parallel to the pulling direction.

6. The connecting device according to any one of claims 1-5, comprising an external actuator (109), distinct from the slider (110) and provided with a pushing surface (PS') extending outside the flange (3),
and wherein the slider (110) includes the retaining surface (RS) and is actuatable to cause the retaining surface (RS) to be displaced, the slider (110) being actuatable by the external actuator (9; 109) to unlock a locked state of the female connecting member (31) when the connector (1) is in connected state with the identical complementary connector (101).

7. The connecting device according to any one of claims 1-5, wherein, the slider (10; 110) is an annular piece that includes the retaining surface (RS) and actuatable by a pushing action, at a side of the slider that is away, preferably opposite, relative to the retaining surface (RS) to cause the retaining surface (RS) to be displaced radially outward, whereby the slider (10; 110) is a locking slider that is actuatable to unlock a locked state of the female connecting member (31) when the connector (1) is in connected state with the identical complementary connector (101).

8. The connecting device according to any one of the preceding claims, wherein the slider (10; 110) is a locking slider slidable upon actuation of an external actuator (9; 109) provided with a pushing surface (PS; PS') extending outside the flange (3), the retaining surface (RS) being provided in a portion (51) of the locking slider which is radially movable toward the longitudinal axis (A2), to engage the fastening region (104r) of the second insert.

9. The connecting device according to any one of the preceding claims, wherein the slider (10; 110) is a locking slider of annular shape, extending around the tubular body 2 and retained in a housing (8) at least partly formed by the flange (3), the locking slider being axially inserted and mounted in the housing (8) by a rear access facing in opposite direction from the flange front face (F3).

10. The connecting device according to any one of the preceding claims, wherein the locking slider is annular, the retaining surface (RS) being provided at an end of the locking slider at the opposite from another end coupled to or provided with an external actuator (9; 109), said another end being pushable along a first direction (Y), which is an unlocking direction opposite to an elastic return force provided by the locking slider.

11. The connecting device according to any one of the preceding claims, wherein the male connecting member (32) forms the only part of the connector (1) protruding axially from the flange front face (F3).

12. The connecting device according to any one of the preceding claims, wherein the slider is configured to be transversely moved, along a first direction perpendicular to the longitudinal axis (A2) upon insertion of the second insert via the axial access (30),
and wherein the locking slider (10) includes an elastic return part (53) in radial contact with the tubular body (2) and providing an elastic return force directed toward a second direction that is a sliding direction opposite to the first direction, whereby a clipping in an inserted state of the second insert in the female connecting member (31) is allowed due to the elastic return force.

13. The connecting device according to any one of the preceding claims when depending on claim 2, wherein the connector (1) is a first aseptic coupling device for coupling to a second aseptic coupling device consisting in the identical complementary connector (101) and an associated membrane, the membrane (M) of the first aseptic coupling device being a first peel off membrane removably coupled to and at least partially covering the flange front face (F3) and the sealing member (J),
and wherein the flange (3) of the connector (1), preferably integrally formed in one piece with the tubular body (2), has an external side wall (18) surrounding a housing (8) or recess forming the slide (80; 80'), the slider being partly or entirely housed in the housing (8) or recess.

14. The connecting device according to claim 12, wherein the external side wall (18) has a radial opening (18c), a protrusion or link passing through the radial opening (18c) to allow driving the slider along the slide (80).

15. The connecting device according to claim 12, wherein the slider is a locking slider (10) clipped inside the flange (3), preferably to be permanently retained in the housing (8),
and wherein the external side wall (18) defines a circumference all around the slider (10) and is configured to prevent the slider to be displaced along the slide (80) by a transverse actuation.

16. An aseptic coupling arrangement (20), comprising a first coupling device and a second coupling device, which are each provided with the connecting device according to any one of the claims 1-12, wherein in a pre-coupled state of the aseptic coupling arrangement, with two membranes (M) interposed between the connector (1) of the first coupling device and the connector (101) of the second coupling device , the flange (3) of the first coupling device and the flange (3) of the second coupling device are interlocked in a flange-to-flange connection area where the first insert and the second insert extend, the connection area being an annular connection area surrounded by:
- a first slider, which is the slider provided in the first aseptic coupling device; and
- a second slider, which is the slider provided in the second aseptic coupling device.

17. The aseptic coupling arrangement according to claim 16, wherein the first slider and the second slider are inverted in position, while both extending perpendicular to the longitudinal axis (A2) to provide a locking of the pre-coupled state.
